# EUROPÄISCHE PATENTSCHRIFT

(11) **EP 4 251 098 B1**
(45) Veröffentlichungstag und Bekanntmachung des Hinweises auf die Patenterteilung: **22.07.2026**
(21) Anmeldenummer: 21814780.9
(22) Anmeldetag: 19.11.2021
(51) Int. Cl.: A61F 2/90, A61F 2/966

(54) **MEDIZINISCHES SET ZUR BEHANDLUNG VON BLUTGEFÄSSERKRANKUNGEN**
MEDICAL KIT FOR TREATMENT OF VASCULAR DISEASES
KIT MÉDICAL POUR LE TRAITEMENT DE MALADIES VASCULAIRES

(30) Priorität: 26.11.2020 DE 202020106807 U
(43) Veröffentlichungstag der Anmeldung: 04.10.2023
(73) Patentinhaber: Acandis GmbH, 75177 Pforzheim (DE)
(72) Erfinder: SCHÜSSLER, Andreas, 76327 Pfinztal (DE); BRASCHKAT, Andrés, 75015 Bretten (DE); DING, Andreas, 76137 Karlsruhe (DE)
(74) Vertreter: Meissner Bolte Partnerschaft mbB
(86) Internationale Anmeldenummer: PCT/EP2021/082253
(87) Internationale Veröffentlichungsnummer: WO 2022/112116

(56) Entgegenhaltungen:
- EP-A1- 1 880 695
- DE-A1- 102012 112 730
- DE-U1- 202013 012 692

## Beschreibung

Die Erfindung betrifft ein medizinisches Set zur Behandlung von Blutgefäßerkrankungen.

Aus der Praxis sind zur Behandlung von Blutgefäßerkrankungen, wie Stenosen oder Aneurysmen, Stents bekannt, die in einem Set mit einer Einführungshilfe, auch "Introducer" genannt, und einem Transportdraht angeboten werden. Der Stent ist üblicherweise in einem komprimierten Zustand auf dem Transportdraht angeordnet und mit dem Transportdraht in der Einführungshilfe positioniert. Die Einführungshilfe bildet im Wesentlichen einen Schlauch oder ein Rohr, das den komprimierten Zustand des Stents aufrechterhält.

Für die eigentliche Behandlung wird der Stent mit dem Transportdraht durch einen Katheter an den Behandlungsort geführt. Dort wird der Stent aus dem Katheter entlassen und faltet sich dabei auf. Bei einigen Behandlungsmethoden kann es erforderlich sein, nach der vollständigen Entlassung des Stents in das Blutgefäß den Katheter in distale Richtung durch den Stent zu führen. Dies kann beispielsweise notwendig sein, um mittels eines Ballons den Stent nochmals aktiv zu expandieren bzw. an die Blutgefäßwand anzudrücken. Andere Gründe für das Einführen des Katheters in den expandierten Stent können darin bestehen, dass ein Blutgefäßareal, das distal des Stents angeordnet ist, zusätzlich behandelt werden soll. Teilweise führen Operateure den Katheter auch durch den expandierten Stent hindurch, um sicherzustellen, dass der Stent ausreichend durchgängig ist.

Bei dieser Prozedur, bei der der Katheter nach distal geschoben und durch den Stent hindurchgeführt wird, besteht das Risiko, dass sich der Katheter an den Drahtenden des Stents verhakt. Je nach Konstruktion des Stents kann es insbesondere vorkommen, dass das proximale Stentende sich nicht ausreichend aufweitet, so dass Drahtenden leicht in das Blutgefäß hineinragen. Wenn nun der Katheter nach distal geschoben wird, kann sich die Katheterspitze an diesen Drahtenden verhaken. Dies kann dazu führen, dass der Stent weiter von der Blutgefäßwand gelöst wird oder durch eine Schiebebewegung, die auf den Stent einwirkt, eine Beschädigung der Blutgefäßwand auftritt. Außerdem besteht das Risiko, dass sich der Katheter nicht durch den Stent schieben lässt, so dass beispielsweise eine Behandlung nachfolgender Blutgefäßareale durch den Katheter nicht mehr möglich ist.

Der bisherige Stand der Technik hat das zuvor beschriebene Problem bislang nicht erkannt. Zwar sind Stents bekannt, die aus Drähten gebildet sind, welche an wenigstens einem Stentende Schlaufen bilden. Solche Stents sind beispielswiese in DE 10 2012 112 730 A1 und DE 10 2015 107 291 A1 beschrieben, wobei darin kein Hinweis enthalten ist, wie ein Verhaken einer Katheterspitze an den Drahtenden eines proximalen Endes des jeweiligen Stents zu vermeiden wäre.

Aus DE 10 2018 333 345 A1 beschreibt einen Stent, der aus einem einzigen Draht gebildet ist, der an beiden Stentenden zu Schlaufen geformt ist. Der bekannte Stent zeichnet sich durch ein besonderes Expansionsverhalten aus. Die oben beschriebene Prozedur, bei welchem der Katheter nach distal durch den expandierten Stent geschoben wird und das damit verbundene Problem wurden jedoch nicht erkannt.

DE 20 2013 012 692 U1 offenbart zwar einen Stent, der aus mehreren Einzeldrähten gebildet sein kann, wobei die Einzeldrähte an einem oder beiden Enden zu Formschlaufen zusammengeschweißt, -geklebt oder verschmolzen werden. Welche Konfiguration des Stents für welche Herausforderung einzusetzen ist, geht daraus jedoch nicht hervor. Zudem ist nicht nur die Herstellung der Formschlaufen aufwändig, sondern die Verbindung der Einzeldrähte insbesondere an den hochbeanspruchten Stentenden birgt das Risiko, dass die Verbindungsstellen aufbrechen und offene Drahtenden entstehen, die zu einer Verletzung von Gefäßwänden führen können.

Aus EP 1 880 695 A1 ist ein System zu Zuführen von Stents bekannt, wobei die Art des Stents nicht näher erläutert wird. Denkbar ist zwar, einen Stent gemäß DE 10 2012 112 730 A1 mit dem System zuzuführen. Die DE 10 2012 112 730 A1 erläutert dazu, dass es besonders vorteilhaft ist, das Stentende mit Schlaufen so nach vorne in Schubrichtung auszurichten, dass es die Reibung beim Vorschieben des Stents durch ein Katheter verringert. Im expandierten Zustand des Stents innerhalb eines Blutgefäßes liegen die freien Drahtenden an einem proximalen Ende, so dass die Gefahr besteht, dass ein Katheter beim Vorschieben durch den Stent mit dem proximalen offen Drahtenden verhakt.

Der Erfindung liegt daher die Aufgabe zu Grunde, ein medizinisches Set zur Behandlung von Blutgefäßerkrankungen anzugeben, das das Risiko eines Verhakens zwischen einer Katheterspitze und einem im Blutgefäß expandierten Stent reduziert und so eine Folgebehandlung mit dem Katheter ermöglicht.

Erfindungsgemäß wird diese Aufgabe durch den Gegenstand des Anspruchs 1 gelöst.

So beruht die Erfindung auf dem Gedanken, ein medizinisches Set zur Behandlung von Blutgefäßerkrankungen anzugeben, wobei das Set ausgestattet ist mit
- einer rohr- oder schlauchförmigen Einführungshilfe,
- einem Transportdraht, der eine distale Spitze aufweist, und
- einem Stent, der ein Gittergeflecht aus mehreren Drähten aufweist, die an einem proximalen Stentende Schlaufen und an einem distalen Stentende offene Drahtenden bilden,
wobei der Stent auf dem Transportdraht und mit diesem innerhalb der Einführungshilfe angeordnet ist, und wobei das distale Längsende des Stents näher als das proximale Längsende an der distalen Spitze des Transportdrahts positioniert ist.

Die Erfindung nutzt zwei Aspekte, um ein Verhaken einer Katheterspitze am proximalen Stentende zu vermeiden.

Einerseits ist bei dem erfindungsgemäßen Set vorteilhaft vorgesehen, dass der Stent an einem Stentende Schlaufen aufweist. Die Schlaufen ermöglichen es durch ihre Krümmung, dass eine Katheterspitze abgelenkt und so in das Innere des Stents geführt wird. Die Katheterspitze kann sich also nicht so leicht verhaken, wie dies beispielsweise bei offenen Drahtenden der Fall ist.

Andererseits ist bei der Erfindung explizit vorgesehen, dass der Stent derart auf dem Transportdraht positioniert ist, dass die Schlaufen an einem proximalen Stentende angeordnet sind. Bei dem Set ist also sichergestellt, dass durch die Anordnung des Transportdrahts mit dem Stent in der Einführungshilfe der Stent nur so in ein Blutgefäß eingesetzt werden kann, dass die Schlaufen im Blutgefäß proximal ausgerichtet sind. Die Schlaufen weiten sich also bei der Entlassung aus einem Katheter als letztes auf und liegen so nahe an der Katheterspitze. Wenn nun der Katheter wieder in den Stent eingeschoben werden soll, so erfolgt dies grundsätzlich von der Seite aus, an der die Schlaufen positioniert sind. So ist das Risiko, dass der Katheter mit dem Stent verhakt, wenn der Katheter nach distal in den Stent eingeschoben wird, deutlich reduziert.

Ein weiterer vorteilhafter Effekt der am proximalen Stentende angeordneten Schlaufen besteht darin, dass durch die Schlaufen die Radialkraft am proximalen Stentende erhöht wird. Die beiden Schenkel einer Schlaufe tendieren nämlich dazu, sich voneinander zu entfernen, also sich aufzuspreizen. Das hat zur Folge, dass sich das proximale Stentende sehr gut aufweitet bzw. sehr gut expandiert. Damit ist das Risiko, dass sich am proximalen Ende des Stents Drähte in das Blutgefäß hineinragen, weil sie nicht ausreichend an der Gefäßwand anliegen, ebenfalls reduziert.

Alle vorgenannten Effekte in der Zusammenschau bewirken, dass nach einer vollständigen Entlassung des Stents ein Katheter gut nach distal durch den Stent geschoben werden kann, wobei die Gefahr eines Verhakens zwischen dem Katheter und dem Stent deutlich reduziert ist.

Um das proximale Stentende bei der Implantation des Stents in ein Blutgefäß gut erkennen zu können, ist bei einer bevorzugten Ausführungsform der Erfindung vorgesehen, dass das proximale Stentende, insbesondere die Schlaufen, zwischen einem proximalen und einem distalen Röntgenmarker des Transportdrahts angeordnet ist. Die Röntgenmarker sind unter Röntgenkontrolle gut erkennbar, so dass der Operateur nachvollziehen kann, wo sich das proximale Stentende derzeit befindet. Dies ist wesentlich, um bei einer Teilentlassung des Stents abschätzen zu können, ob die endgültige Position des Stents korrekt ist. Sobald die Positionierung abgeschlossen ist, kann dann der Stent vollständig aus dem Katheter entlassen werden.

Vorzugsweise weist der Transportdraht einen Transportabschnitt und einen Führungsabschnitt auf. Der Führungsabschnitt kann einen Durchmesser aufweisen, der größer als ein Durchmesser des Transportabschnitts ist. Ferner kann der Führungsabschnitt eine Anschlagfläche aufweisen, an der die Schlaufen des proximalen Stentendes längsaxial anliegen. Die Anschlagfläche ermöglicht es so, den Stent durch die Einführungshilfe und/oder den Katheter zu schieben. Dazu liegen die Schlaufen längsaxial an der Anschlagfläche an. Bevorzugt ist es außerdem, wenn der Führungsabschnitt des Transportdrahts einen Querschnittsdurchmesser aufweist, der im Wesentlichen einem Innendurchmesser der Einführungshilfe und/oder eines Katheters entspricht. So ist sichergestellt, dass der Stent in dem an dem Führungsabschnitt anschließenden Transportabschnitt verbleibt. Insbesondere kann so verhindert werden, dass der Stent sich zwischen einer Innenwand der Einführungshilfe und/oder des Katheters und dem Transportdraht positioniert, was dazu führen könnte, dass der Stent durch den Transportdraht nicht mehr transportiert wird.

Bei einer bevorzugten Variante der Erfindung ist der proximale Röntgenmarker des Transportdrahts an einem distalen Ende des Führungsabschnitts angeordnet. Ferner kann der proximale Röntgenmarker unmittelbar an die Anschlagfläche angrenzen und/oder die Anschlagfläche bilden. Diese Anordnung des proximalen Röntgenmarkers stellt sicher, dass ein Operateur das proximale Stentende unter Röntgenkontrolle gut erkennt. Insbesondere ist so die Entlassung des Stents in ein Blutgefäß besonders gut steuerbar, da dem Operateur die Information vorliegt, wie sich das proximale Stentende der Katheterspitze nähert.

Der Transportabschnitt des Transportdrahts erstreckt sich vorzugsweise vollständig durch den Stent. Die distale Spitze des Transportdrahts kann Teil des Transportabschnitts sein. Vorzugsweise ist die distale Spitze flexibel, insbesondere flexibler als der Rest des Transportabschnitts. So kann die distale Spitze, die vorzugsweise über den Stent hinaus vorsteht, gut durch gekrümmte Blutgefäße navigieren.

Eine bevorzugte Variante der Erfindung sieht vor, dass der distale Röntgenmarker des Transportdrahts im Transportabschnitt angeordnet ist. Insbesondere kann der distale Röntgenmarker zwischen der distalen Spitze und der Anschlagfläche des Führungsabschnitts angeordnet sein. Der distale Röntgenmarker weist vorzugsweise einen Außendurchmesser auf, der kleiner als der Außendurchmesser des Führungsabschnitts ist, so dass sich zwischen dem distalen Röntgenmarker und der Innenwand der Einführungshilfe ein Spalt bildet, durch welchen sich das Gittergeflecht des Stents erstrecken kann. Vorzugsweise ist der distale Röntgenmarker nahe am proximalen Ende des Stents positioniert. Es ist besonders vorteilhaft, wenn die Schlaufen des proximalen Stentendes zwischen dem proximalen Röntgenmarker und dem distalen Röntgenmarker des Transportdrahts angeordnet sind. So ist die Position der Schlaufen für einen Operateur unter Röntgenkontrolle besonders gut erkennbar.

Der Transportdraht, insbesondere der Führungsabschnitt, kann in einer bevorzugten Variante der Erfindung proximal aus der Einführungshilfe vorstehen und einen proximalen Greifabschnitt mit einer Riffelung aufweisen. Die Gestaltung des Greifabschnitts ist vorteilhaft, weil auf diese Weise der Transportdraht für einen Operateur gut zu greifen ist. Ein guter Kontakt mit dem Transportdraht ist zweckmäßig, um den Transportdraht gut durch einen Katheter und ein Blutgefäß führen zu können. Durch die Riffelung wird dabei vermieden, dass der Operateur entlang des Transportdrahts abrutscht. Insofern verbessert die Riffelung im Greifabschnitt die Navigierbarkeit des Transportdrahts.

Bei einer weiteren bevorzugten Ausführungsform der Erfindung ist vorgesehen, dass die Einführungshilfe an einem distalen Ende einen Anschluss zur Verbindung mit einem Katheter aufweist. Die Einführungshilfe ist also direkt an einen Katheter anschließbar. Auf diese Weise kann der Stent mit dem Transportdraht einfach von der Einführungshilfe in einen Katheter überführt werden. Durch den Katheter gelangt der Stent, der weiterhin auf dem Transportdraht angeordnet ist, in das zu behandelnde Blutgefäß.

Bei einer weiteren bevorzugten Ausführungsform der Erfindung sind am proximalen Stentende des Stents erste Schlaufen und zweite Schlaufen angeordnet. Die ersten Schlaufen können längsaxial über die zweiten Schlaufen vorstehen. Insbesondere können am proximalen Stentende die gleiche Anzahl von ersten Schlaufen und zweiten Schlaufen vorgesehen sein. Bevorzugt ist es, wenn die ersten Schlaufen und die zweiten Schlaufen in Umfangsrichtung abwechselnd angeordnet sind.

Im Wesentlichen ist also bei dieser Ausführungsform der Erfindung vorgesehen, dass am proximalen Stentende abwechselnd längere und kürzere Schlaufen angeordnet sind. Dabei wechselt sich jeweils eine längere Schlaufe mit einer kürzeren Schlaufe ab. Die längeren Schlaufen werden im Rahmen der vorliegenden Anmeldung als erste Schlaufen und die kürzeren Schlaufen als zweite Schlaufen bezeichnet. Die ersten Schlaufen liegen bei dem Set vorzugsweise an der Anschlagfläche an. Die versetzte Anordnung der Schlaufen hat den Vorteil, dass auf diese Weise der Stent auch am proximalen Stentende gut auf einen kleinen Querschnittsdurchmesser komprimierbar ist. Außerdem erleichtert dies ebenfalls das Wiedereinführen eines Katheters in den expandierten Stent. Das Risiko, dass sich der Katheter an dem proximalen Stentende verhakt, wird somit weiter reduziert.

Um es zu ermöglichen, dass der Stent unter Röntgenkontrolle sichtbar ist, ist bevorzugt vorgesehen, dass die Drähte des Gittergeflechts einen Kern aus einem röntgensichtbaren Material und eine Ummantelung aus einem Formgedächtniswerkstoff aufweisen. Der röntgensichtbare Kern ermöglicht es, dass das gesamte Gittergeflecht unter Röntgenkontrolle erkennbar ist. Damit ist nicht nur die Position des Gittergeflechts erkennbar, sondern auch zu sehen, ob das Gittergeflecht bzw. der Stent ausreichend dicht an der Gefäßwand anliegt. Die Ummantelung aus dem Formgedächtniswerkstoff ermöglicht eine Selbstexpansionsfähigkeit. Der Stent weitet sich also nach der Entlassung aus einem Katheter selbsttätig auf und behält vorzugsweise die aufgeweitete Form bei. Vorzugsweise wird eine Nickel-Titan-Legierung als Formgedächtniswerkstoff eingesetzt. Das röntgensichtbare Material für den Kern kann Platin, Gold oder Tantal umfassen. Besonders bevorzugt ist eine Platin-Iridium-Legierung.

Um eine ausreichend hohe Röntgensichtbarkeit zu gewährleisten, ist bei einer weiteren Variante der Erfindung vorgesehen, dass mindestens 20 Vol.-%, insbesondere mindestens 25 Vol.-%, insbesondere mindestens 30 Vol.-% jedes Drahts durch den Kern gebildet sind. Vorzugsweise sind höchstens 40 % des Drahts durch den Kern gebildet.

Bei einer Ausführungsform der Erfindung kann vorgesehen sein, dass der Stent keine zusätzlichen Röntgenmarker aufweist und/oder ausschließlich aus Drähten besteht. Damit wird vermieden, dass durch zusätzliche Bauelemente die Eigenschaften des Gittergeflechts beeinträchtigt werden. Insbesondere Röntgenmarker, die auf die Drähte aufgecrimpt sind, erschweren die Zuführung eines Stents durch einen Katheter, da derartige Röntgenmarker an der Katheterinnenwand reiben können. Durch den Verzicht auf derartige zusätzliche Elemente wird also die Zuführbarkeit des Stents verbessert.

Alternative Ausführungsformen der Erfindung, bei welchen der Stent zusätzliche Röntgenmarker aufweist, sind allerdings nicht ausgeschlossen. Beispielsweise können am proximalen Stentende, insbesondere an den Schlaufen mehrere, insbesondere drei, zusätzliche Röntgenmarker angeordnet sein, um das proximale Stentende unter Röntgenkontrolle hervorzuheben.

In einer besonders bevorzugten Variante der Erfindung weist der Stent des Sets ein Gittergeflecht aus Drähten auf, deren Durchmesser 30 µm beträgt. Vorzugsweise umfasst das Gittergeflecht 24 Drähte, wobei jeder Draht jeweils eine Schlaufe bildet. Am proximalen Stentende sind folglich 24 Schlaufen vorgesehen. Wegen der durch Bildung der Schlaufen zurückgeführten Drähte sind über den Umfang des Gittergeflechts daher 48 Drahtsegmente effektiv wirksam. Vorzugsweise liegen nur am proximalen Stentende Schlaufen vor. Am distalen Stentende bilden die Drähte offene Drahtenden aus. Das Gittergeflecht weist vorzugsweise einen zylindrischen Geflechtabschnitt auf, der das distale Stentende umfasst und sich bis zum proximalen Stentende erstreckt. Das proximale Stentende ist somit nicht mehr Teil des zylindrischen Geflechtabschnitts, das distale Stentende hingegen schon. Im zylindrischen Geflechtabschnitt kann ein Flechtwinkel zwischen 70° und 75° eingestellt sein. Am proximalen Stentende, d.h. beim Übergang zu den Schlaufen, kann der Flechtwinkel kleiner sein. Insbesondere kann der Flechtwinkel am proximalen Stentende 60° betragen. Der Flechtwinkel ist derjenige Winkel, der sich zwischen einer Projektion der Längsachse des Gittergeflechts auf die Wandungsebene, in der die Drähte geflochten sind, und einem der Drähte einstellt. Das proximale Stentende kann überdies konisch aufgefächert sein, d.h. im expandierten, kraftunbelasteten Ruhezustand weiten sich die Schlaufen am proximalen Stentende auf, so dass die Scheitelbögen der Schlaufen auf einem Querschnittsdurchmesser angeordnet sind, der größer als der Querschnittsdurchmesser des zylindrischen Geflechtabschnitts ist. Der Stent kann in dieser Variante insbesondere einen Nenndurchmesser, d.h. einen Durchmesser, den der Stent im implantierten Zustand einnimmt, von 3,5 mm oder 3,0 mm oder 2,5 mm aufweisen. Im kraftunbelasteten, selbstexpandierten Ruhezustand ist der Durchmesser vorzugsweise etwas größer ("Oversizing"), so dass sich der Stent im implantierten Zustand gut gegen eine Gefäßwand abstützt. Vorzugsweise umfasst ein Stent mit einem Nenndurchmesser von 2,5 mm einen Querschnittsdurchmesser im Ruhezustand, der etwa 2,62 mm beträgt. Bei einem Stent mit einem Nenndurchmesser von 3,0 mm beträgt der Querschnittsdurchmesser im Ruhezustand etwa 3,12 mm und bei einem Stent mit einem Nenndurchmesser von 3,5 mm beträgt der Querschnittsdurchmesser im Ruhezustand etwa 3,62 mm. Der Querschnittsdurchmesser im Ruhezustand bezieht sich dabei auf den zylindrischen Geflechtabschnitt des Stents.

Besonders bevorzugt sind folgende Dimensionen des Stents eines erfindungsgemäßen Sets:

| **Ausführungsform** | **Stent-durchmesser** | **Anzahl der Schlaufen (Drähte)** | **Drahtdurchmesser** | **Flechtwinkel** | | **GesamtLänge** | **Länge des proximalen Stentendes** | **Länge des zylindrischen Geflechtabschnitts** |
|---|---|---|---|---|---|---|---|---|
| | OD Stent / mm (±0,2mm) | nl (nw) / - | d / mm (Toleranz nach Spezifikation) | FW-L3 / ° (±2°) | FW.L2 / ° (±4°) | L1 / mm (±1mm) | L2 / mm (±1mm) | L3 / mm (±1mm) |
| 1 | 2,62 | 24 (48) | 0,03 | 70 | 60 | 8,0 | 3,0 | 5,0 |
| 2 | 2,62 | 24 (48) | 0,03 | 70 | 60 | 12,0 | 3,0 | 9,0 |
| 3 | 2,62 | 24 (48) | 0,03 | 70 | 60 | 15,0 | 3,0 | 12,0 |
| 4 | 2,62 | 24 (48) | 0,03 | 70 | 60 | 19,0 | 3,0 | 16,0 |
| 5 | 3,12 | 24 (48) | 0,03 | 75 | 60 | 8,0 | 3,0 | 5,0 |
| 6 | 3,12 | 24 (48) | 0,03 | 75 | 60 | 11,0 | 3,0 | 8.0 |
| 7 | 3,12 | 24 (48) | 0,03 | 75 | 60 | 14,0 | 3,0 | 11,0 |
| 8 | 3,12 | 24 (48) | 0,03 | 75 | 60 | 18,0 | 3,0 | 15,0 |
| 9 | 3,62 | 24 (48) | 0,03 | 75 | 60 | 8,0 | 3,5 | 4,5 |
| 10 | 3,62 | 24 (48) | 0,03 | 75 | 60 | 12,0 | 3,5 | 8,5 |
| 11 | 3,62 | 24 (48) | 0,03 | 75 | 60 | 15,0 | 3,5 | 11,5 |
| 12 | 3,62 | 24 (48) | 0,03 | 75 | 60 | 19,0 | 3,5 | 15,5 |

Die Erfindung wird im Folgenden anhand eines Ausführungsbeispiels unter Bezugnahme auf die beigefügten, schematischen Zeichnungen näher erläutert. Darin zeigen
- Fig. 1: eine Längsschnittansicht durch ein erfindungsgemäßes medizinisches Set nach einem bevorzugten Ausführungsbeispiel;
- Fig. 2: einen Längsschnitt durch einen Katheter mit dem Transportdraht und dem Stent aus dem Set gemäß Fig. 1;
- Fig. 3: eine Längsschnittansicht durch ein Blutgefäß beim Einsetzen des Stents aus dem Set gemäß Fig. 1;
- Fig. 4: eine Seitenansicht eines Stents aus dem Set gemäß Fig. 1; und
- Fig. 5: eine Seitenansicht des Stents gemäß Fig. 4 und einer Katheterspitze vor dem Wiedereinführen der Katheterspitze in den Stent.

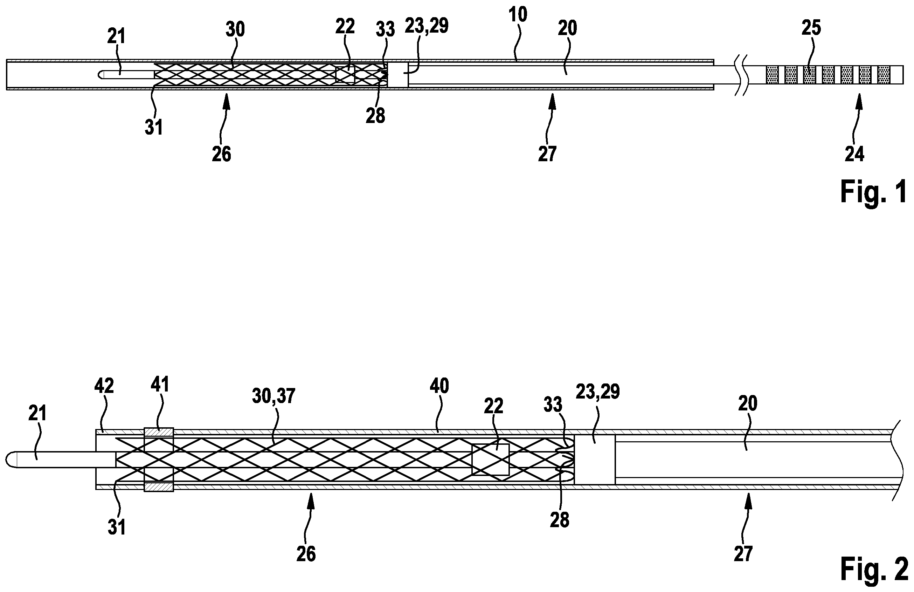

Fig. 1 zeigt ein erfindungsgemäßes Set, wobei die Einführungshilfe 10 in einem Längsschnitt dargestellt ist. In der Einführungshilfe 10 ist ein Transportdraht 20 angeordnet. Der Transportdraht umfasst einen Führungsabschnitt 27 und einen Transportabschnitt 26. Im Transportabschnitt ist ein Stent 30 positioniert. Der Transportabschnitt steht über den Stent nach distal vor und bildet dort eine distale Spitze 21.

Die distale Spitze 21 ist Teil des Transportabschnitts 26, der einen kleineren Querschnittsdurchmesser als der proximal angeordnete Führungsabschnitt 27 aufweist. Für den Übergang zwischen dem Transportabschnitt 26 und dem Führungsabschnitt 27 ist die Anschlagfläche 28 vorgesehen. Die Anschlagfläche erstreckt sich im Wesentlichen ringförmig um den Transportabschnitt 26. Direkt an die Anschlagfläche 28 schließt sich ein proximaler Röntgenmarker 23 des Transportdrahts 20 an. Der Röntgenmarker 23 kann auch die Anschlagfläche bilden.

Am proximalen Transportdrahtende 24 weist der Transportdraht 20 einen Greifabschnitt 25 auf. Der Greifabschnitt 25 umfasst eine Riffelung, so dass der Transportdraht durch einen Operateur gut gegriffen werden kann.

Wie in Fig. 1 ebenfalls erkennbar ist, ist lediglich ein distaler Abschnitt des Transportdrahts 20 mit dem Stent 30 innerhalb der Einführungshilfe 10 angeordnet. Die Einführungshilfe 10 kann einen Kunststoffschlauch oder ein Kunststoffrohr aufweisen. Vorzugsweise ist die Einführungshilfe transparent.

Nicht dargestellt, jedoch bevorzugt vorhanden, ist ein Anschlusselement am distalen Ende der Einführungshilfe, wobei mit dem Anschlusselement eine direkte Verbindung zwischen der Einführungshilfe und einem Katheter hergestellt werden kann. Vorzugsweise wird ein Katheter eingesetzt, dessen Innendurchmesser dem Innendurchmesser der Einführungshilfe entspricht. So ist gewährleistet, dass der Stent im komprimierten Zustand von der Einführungshilfe 10 in einen Katheter 40 überführt werden kann.

Fig. 1 zeigt ebenfalls, dass das proximale Stentende 33 vorzugsweise zwischen zwei Röntgenmarkern 22, 23 des Transportdrahts 20 angeordnet ist. Dabei kann der distale Röntgenmarker 22 so gestaltet sein, dass er radial nach außen vorstehende Vorsprünge aufweist, die in das Gittergeflecht des Stents 30 eingreifen. Auf diese Weise kann der Stent nicht nur über den Transportdraht 20 nach distal an einen Behandlungsort geführt werden. Vielmehr ist es auch möglich, einen teilentlassenen Stent 30 mittels des Transportdrahts 20 wieder in den Katheter 40 zurückzuziehen.

Die Zuführung des Stents 30 durch den Katheter 40 ist in Fig. 2 gezeigt. Darin ist erkennbar, dass der Katheter, der in einer Längsschnittansicht dargestellt ist, an einem distalen Ende einen distalen Kathetermarker 41 aufweist. Es können grundsätzlich auch mehrere distale Kathetermarker 41 vorgesehen sein. Der Kathetermarker 41 umfasst vorzugsweise ein röntgensichtbares Material. Anhand der Lage der Röntgenmarker 22, 23 und des Kathetermarkers 41 ist es für einen Operateur leicht möglich, die exakte Position des Stents 30 zu erkennen und ggf. zu justieren.

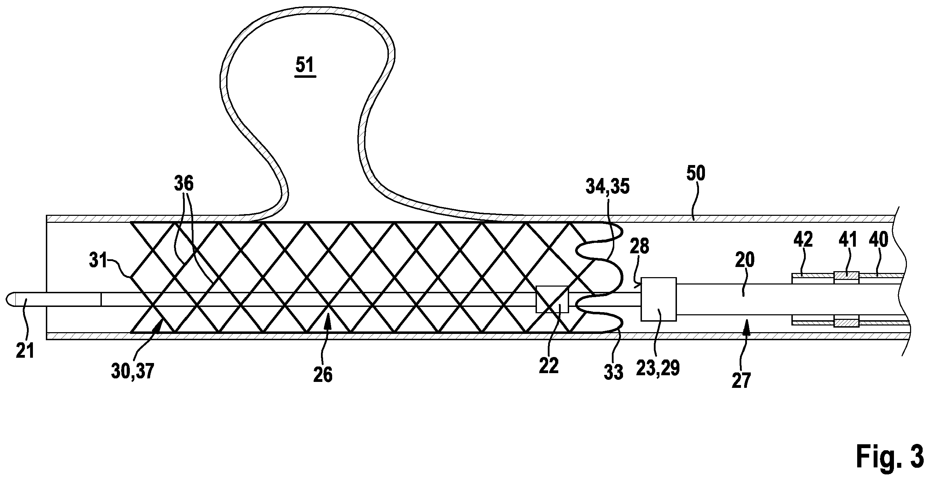

In Fig. 3 ist im Längsschnitt ein Blutgefäß 50 gezeigt, in dem sich ein Aneurysma 51 ausgebildet hat. Um das Aneurysma von einer Blutströmung weitgehend abzuschirmen, wird das medizinische Set eingesetzt. Dabei wird der Stent 30 mittels des Transportdrahts 20 über einen Katheter 40 an den Behandlungsort geführt. Anschließend wird der Stent 30 aus dem Katheter 40 entlassen. Dadurch weitet sich der Stent 30 auf und legt sich an die Gefäßwand an. Der Stent überspannt so einen Hals eines Aneurysmas 51 und bewirkt, dass der Blutstrom in das Aneurysma 51 reduziert wird. Dies führt zur Bildung von Thromben in dem Aneurysma 51, so dass dieses im Wesentlichen verödet.

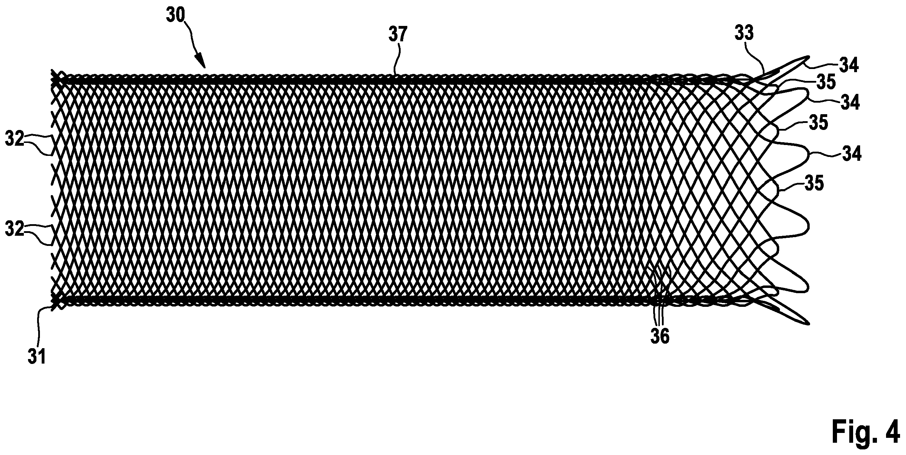

In Fig. 4 ist eine Seitenansicht eines Stents 30 gezeigt. Zur besseren Übersichtlichkeit ist jedoch eine hintere Hälfte des Stents 30 ausgeblendet. Der Stent 30 weist im expandierten Zustand vorzugsweise einen Außendurchmesser zwischen 2,5 mm und 3,5 mm auf.

Der Stent 30 weist ein Gittergeflecht 37 auf, das aus Drähten 36 gebildet ist. Die Drähte 36 umfassen vorzugsweise jeweils einen Kern aus einem röntgensichtbaren Material und eine Ummantelung aus einem Formgedächtniswerkstoff. Der Stent 30 weist vorzugsweise 48 Drähte auf. Die Drähte 36 sind am distalen Stentende 31 offen bzw. bilden offene Drahtenden 32. Am proximalen Stentende 33 sind die Drähte 36 jedoch jeweils umgelenkt und bilden Schlaufen 34, 35. Erkennbar ist in Fig. 4 außerdem, dass der Stent 30 am proximalen Stentende 33 vorzugsweise konisch aufgeweitet ist. Damit ist zusätzlich sichergestellt, dass das proximale Stentende 33 gut expandiert und sich eng an eine Gefäßwand anlegt.

Der Stent 30 weist am proximalen Stentende 33 erste Schlaufen 34 und zweite Schlaufen 35 auf. Die ersten Schlaufen 34 stehen längsaxial über die zweiten Schlaufen 35 vor. Mit anderen Worten bilden die ersten Schlaufen 34 lange Schlaufen, wogegen die zweiten Schlaufen 35 kurze Schlaufen bilden. Die ersten Schlaufen 34 und die zweiten Schlaufen 35 wechseln sich regelmäßig ab. In Umfangsrichtung folgt also auf jede erste Schlaufe 34 eine zweite Schlaufe 35, woraufhin wieder eine erste Schlaufe 34 und anschließend eine zweite Schlaufe 35 folgt. Folglich ist jede zweite Schlaufe in Umfangsrichtung eine erste Schlaufe 34 bzw. lange Schlaufe.

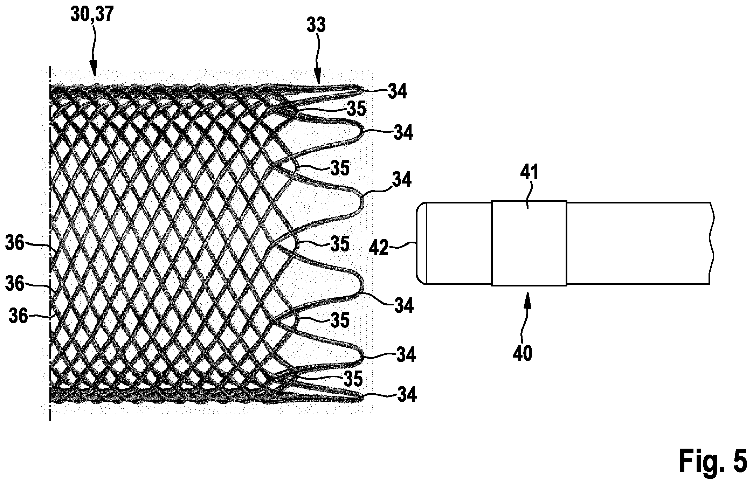

In Fig. 5 ist die Funktionsweise der Erfindung besonders gut erkennbar. Bei dem hier beschriebenen medizinischen Set ist der Stent 30 so in der Einführungshilfe 10 angeordnet, dass die Schlaufen 34, 35 des Stents 30 am proximalen Stentende 33 angeordnet sind. Nach der Entlassung des Stents 30 aus dem Katheter 40 weisen die Schlaufen 34, 35 also in Richtung der Katheterspitze 42. Dies ist in Fig. 5 gezeigt.

Dabei ist darauf hinzuweisen, dass in Fig. 5 die konische Aufweitung des proximalen Stentendes 33 nicht erkennbar ist, da die Form des Stents 30 im implantierten Zustand gezeigt ist. Während in Fig. 4 der Ruhezustand des Stents 30 gezeigt ist, also der Stent ohne äußere Krafteinwirkung vollständig expandiert ist, zeigt Fig. 5 den Zustand, den der Stent 30 in einem Blutgefäß einnimmt. Dabei wirkt die Blutgefäßwand als Widerstand gegen das Aufweiten des Stents 30, so dass sich das proximale Stentende 33 nicht konisch aufweitet. Vielmehr liegt das proximale Stentende 33 dicht an einer Gefäßwand an.

In Fig. 5 ist deutlich erkennbar, dass durch die Schlaufen 34, 35 am proximalen Stentende 33 die Gefahr deutlich reduziert ist, dass der Katheter 40 an dem Gittergeflecht 37 verhakt, wenn der Katheter 40 in distale Richtung bewegt wird. Der Katheter 40 bzw. die Katheterspitze 42 gleitet, sofern sie überhaupt entlang der Gefäßwand an das Gittergeflecht 37 gelangt, an den Schlaufen 34, 35 ab und wird so in das Innere des Stents 30 geführt.

Es wird darauf hingewiesen, dass in allen beigefügten Zeichnungen Elemente auf der linken Seite der Zeichnungsfläche "distal" und Elemente auf der rechten Seite der Zeichnungsfläche "proximal" angeordnet sind.

### Bezugszeichenliste

- 10: Einführungshilfe (Introducer)
- 20: Transportdraht
- 21: distale Spitze
- 22: distaler Röntgenmarker
- 23: proximaler Röntgenmarker
- 24: proximales Transportdrahtende
- 25: Greifabschnitt
- 26: Transportabschnitt
- 27: Führungsabschnitt
- 28: Anschlagfläche
- 29: distales Ende des Führungsabschnitts 27
- 30: Stent
- 31: distales Stentende
- 32: offenes Drahtende
- 33: proximales Stentende
- 34: erste Schlaufe
- 35: zweite Schlaufe
- 36: Draht
- 37: Gittergeflecht
- 40: Katheter
- 41: distaler Kathetermarker
- 42: Katheterspitze
- 50: Blutgefäß
- 51: Aneurysma

## Patentansprüche

1. Medizinisches Set zur Behandlung von Blutgefäßerkrankungen mit
- einer rohr- oder schlauchförmigen Einführungshilfe (10),
- einem Transportdraht (20), der eine distale Spitze (21) aufweist und
- einem Stent (30), der ein Gittergeflecht (37) aus mehreren Drähten (36) aufweist, die an einem proximalen Stentende (33) Schlaufen (34, 35) und an einem distalen Stentende (31) offene Drahtenden (32) bilden,
wobei der Stent (30) auf dem Transportdraht (20) und mit diesem innerhalb der Einführungshilfe (10) angeordnet ist, und wobei das distale Stentende (31) näher an der distalen Spitze (21) des Transportdrahts (20) positioniert ist als das proximale Stentende (33).

2. Medizinisches Set nach Anspruch 1,
**dadurch gekennzeichnet, dass**
das proximale Stentende (33), insbesondere die Schlaufen (34, 35), zwischen einem proximalen und einem distalen Röntgenmarker (22, 23) des Transportdrahts (20) angeordnet ist.

3. Medizinisches Set nach Anspruch 1 oder 2,
**dadurch gekennzeichnet, dass**
der Transportdraht (20) einen Transportabschnitt (26) und einen Führungsabschnitt (27) mit einem Durchmesser aufweist, der größer als ein Durchmesser des Transportabschnitts (26) ist, wobei der Führungsabschnitt (27) eine Anschlagfläche (28) aufweist, an der die Schlaufen (34, 35) des proximalen Stentendes (33) längsaxial anliegen können.

4. Medizinisches Set nach Anspruch 3,
**dadurch gekennzeichnet, dass**
der proximale Röntgenmarker (23) des Transportdrahts (20) an einem distalen Ende (29) des Führungsabschnitts (27) angeordnet ist.

5. Medizinisches Set nach Anspruch 3 oder 4,
**dadurch gekennzeichnet, dass**
der proximale Röntgenmarker (23) unmittelbar an die Anschlagfläche (28) angrenzt und/oder die Anschlagfläche (28) bildet.

6. Medizinisches Set nach einem der Ansprüche 3 bis 5,
**dadurch gekennzeichnet, dass**
sich der Transportabschnitt (26) des Transportdrahts (20) vollständig durch den Stent (30) erstreckt.

7. Medizinisches Set nach einem der Ansprüche 3 bis 6,
**dadurch gekennzeichnet, dass**
der distale Röntgenmarker (22) des Transportdrahts (20) im Transportabschnitt (26) angeordnet ist.

8. Medizinisches Set nach einem der vorhergehenden Ansprüche,
**dadurch gekennzeichnet, dass**
der Transportdraht (20), insbesondere der Führungsabschnitt (27), proximal aus der Einführungshilfe (10) vorsteht und einen proximalen Greifabschnitt (25) mit einer Riffelung aufweist.

9. Medizinisches Set nach einem der vorhergehenden Ansprüche,
**dadurch gekennzeichnet, dass**
die Einführungshilfe (10) an ihrem distalen Ende einen Anschluss zur Verbindung mit einem Katheter (40) aufweist.

10. Medizinisches Set nach einem der vorhergehenden Ansprüche,
**dadurch gekennzeichnet, dass**
am proximalen Stentende (33) des Stents (30) erste Schlaufen (34) und zweite Schlaufen (35) angeordnet sind, wobei die ersten Schlaufen (34) längsaxial über die zweiten Schlaufen (35) vorstehen.

11. Medizinisches Set nach Anspruch 10,
**dadurch gekennzeichnet, dass**
am proximalen Stentende (33) die gleiche Anzahl von ersten Schlaufen (34) und zweiten Schlaufen (35) vorgesehen ist, wobei die ersten Schlaufen (34) und die zweiten Schlaufen (35) in Umfangsrichtung abwechselnd angeordnet sind.

12. Medizinisches Set nach einem der vorhergehenden Ansprüche,
**dadurch gekennzeichnet, dass**
die Drähte (36) des Gittergeflechts (37) einen Kern aus einem röntgensichtbaren Material und eine Ummantelung aus einem Formgedächtniswerkstoff aufweisen.

13. Medizinisches Set nach Anspruch 12,
**dadurch gekennzeichnet, dass**
mindestens 10 vol.-%, insbesondere mindestens 20 vol.-%, insbesondere mindestens 25 vol.-%, insbesondere mindestens 30 vol.-% jedes Drahts (36) durch den Kern gebildet sind.

14. Medizinisches Set nach einem der vorhergehenden Ansprüche,
**dadurch gekennzeichnet, dass**
der Stent (30) keine zusätzlichen Röntgenmarker aufweist und/oder ausschließlich aus Drähten (36) besteht.

## Claims

1. Medical kit for the treatment of vascular diseases, with
- a tubular or hose-like insertion aid (10),
- a transporting wire (20) that comprises a distal tip (21) and
- a stent (30) that comprises a lattice mesh (37) made of several wires (36), which at a proximal stent end (33) forms loops (34, 35) and at a distal stent end (31) forms open wire ends (32),
wherein the stent (30) is arranged on the transporting wire (20) and together with the latter is arranged within the insertion aid (10), and wherein the distal stent end (31) is positioned closer to the distal tip (21) of the transporting wire (20) than the proximal stent end (33).

2. Medical kit according to claim 1,
**characterised in that**
the proximal stent end (33), more particularly the loops (34, 35), is arranged between a proximal and a distal X-ray marker (22, 23) of the transporting wire (20).

3. Medical kit according to claim 1 or 2,
**characterised in that**
the transporting wire (20) comprises a transporting section (26) and a guiding section (27) with a diameter that is greater than a diameter of the transporting section (26), wherein the guiding section (27) has a stop surface (28) onto which the loops (34, 35) of the proximal stent end (33) can abut in a longitudinal axial manner.

4. Medical kit according to claim 3,
**characterised in that**
the proximal X-ray marker (23) of the transporting wire (20) is arranged at a distal end (29) of the guiding section (27).

5. Medical kit according to claim 3 or 4,
**characterised in that**
the proximal X-ray marker (23) is directly adjacent to the stop surface (28) and/or forms the stop surface (28).

6. Medical kit according to any one of claims 3 to 5,
**characterised in that**
the transporting section (26) of the transporting wire (20) extends completely through the stent (30).

7. Medical kit according to any one of claims 3 to 6,
**characterised in that**
the distal X-ray marker (22) of the transporting wire (20) is arranged in the transporting section (26).

8. Medical kit according to any one of the preceding claims,
**characterised in that**
the transporting wire (20), more particularly the guiding section (27) protrudes proximally from the insertion aid (10) and comprises a proximal grip section (25) with ribbing.

9. Medical kit according to any one of the preceding claims,
**characterised in that**
at its distal end, the insertion aid (10) has a connection piece for connecting to a catheter (40).

10. Medical kit according to any one of the preceding claims
**characterised in that**
at the proximal stent end (33) of the stent (30), first loops (34) and second loops (35) are arranged, wherein the first loops (34) project beyond the second loops (35) in a longitudinal axial manner.

11. Medical kit according to claim 10,
**characterised in that**
at the proximal stent end (33), the same number of first loops (34) and second loops (35) are provided, wherein the first loops (34) and the second loops (35) are arranged in an alternating manner in the circumferential direction.

12. Medical kit according to any one of the preceding claims.
**characterised in that** the wires (36) of the lattice mesh (37) have a core made of an X-ray visible material and a sheathing made of a shape-memory material.

13. Medical kit according to claim 12,
**characterised in that**
at least 10 vol.%, more particularly at least 20 vol.%, more particularly at least 25 vol.%, more particularly at least 30 vol.% of each wire (36) is formed by the core.

14. Medical kit according to any one of the preceding claims,
**characterised in that**
the stent (30) does not comprise any additional X-ray markers and/or exclusively consists of wires (36).

## Revendications

1. Kit médical pour le traitement des maladies vasculaires, comportant
- une aide à l'insertion tubulaire ou en forme de tube (10),
- un fil de transport (20) ayant une pointe distale (21) et
- un stent (30) qui comporte un treillis (37) composé de plusieurs fils (36) formant des boucles (34, 35) à une extrémité proximale de stent (33) et des extrémités de fil ouvertes (32) à une extrémité distale de stent (31),
le stent (30) étant placé sur le fil de transport (20) et avec celui-ci à l'intérieur de l'aide à l'insertion (10), et l'extrémité distale de stent (31) étant positionnée plus près de la pointe distale (21) du fil de transport (20) que l'extrémité proximale de stent (33) .

2. Kit médical selon la revendication 1, **caractérisé en ce que** l'extrémité proximale de stent (33), en particulier les boucles (34, 35), est placée entre un marqueur radiographique proximal et un distal (22, 23) du fil de transport (20).

3. Kit médical selon la revendication 1 ou 2, **caractérisé en ce que**
le fil de transport (20) comporte une section de transport (26) et une section de guidage (27) d'un diamètre qui est supérieur à un diamètre de la section de transport (26), la section de guidage (27) ayant une surface de butée (28) sur laquelle les boucles (34, 35) de l'extrémité proximale de stent (33) peuvent reposer dans le sens axial longitudinal.

4. Kit médical selon la revendication 3, **caractérisé en ce que** le marqueur radiographique proximal (23) du fil de transport (20) est placé à une extrémité distale (29) de la section de guidage (27).

5. Kit médical selon la revendication 3 ou 4,
**caractérisé en ce que**
le marqueur radiographique proximal (23) est directement adjacent à la surface de butée (28) et/ou forme la surface de butée (28).

6. Kit médical selon l'une des revendications 3 à 5,
**caractérisé en ce que**
la section de transport (26) du fil de transport (20) s'étend complètement à travers le stent (30).

7. Kit médical selon l'une des revendications 3 à 6,
**caractérisé en ce que**
le marqueur radiographique distal (22) du fil de transport (20) est placé dans la section de transport (26) .

8. Kit médical selon l'une des revendications précédentes,
**caractérisé en ce que**
le fil de transport (20), en particulier la section de guidage (27), est proéminent proximalement de l'aide à l'insertion (10) et présente une section de préhension proximale (25) avec un rainurage.

9. Kit médical selon l'une des revendications précédentes,
**caractérisé en ce que**
l'aide à l'insertion (10) présente à son extrémité distale une connexion à un cathéter (40).

10. Kit médical selon l'une des revendications précédentes, **caractérisé en ce que**,
à l'extrémité proximale de stent (33) du stent (30), les premières boucles (34) et les secondes boucles (35) sont disposées, les premières boucles (34) faisant saillie dans le sens axial longitudinal sur les secondes boucles (35).

11. Kit médical selon la revendication 10,
**caractérisé en ce que**,
à l'extrémité proximale de stent (33), le même nombre de premières boucles (34) et de secondes boucles (35) est prévu, les premières boucles (34) et les secondes boucles (35) étant disposées alternativement dans le sens circonférentiel.

12. Kit médical selon l'une des revendications précédentes, **caractérisé en ce que**
les fils (36) du treillis (37) ont un noyau en matériau visible par radiographie et un revêtement en matériau à mémoire de forme.

13. Kit médical selon la revendication 12,
**caractérisé en ce**
**qu'**au moins 10 % en volume, en particulier au moins 20 % en volume, en particulier au moins 25 % en volume, en particulier au moins 30 % en volume de chaque fil (36) sont formés par le noyau.

14. Kit médical selon l'une des revendications précédentes,
**caractérisé en ce que**
le stent (30) ne comporte pas de marqueurs radiographiques supplémentaires et/ou se compose exclusivement de fils (36).
